(19)

**Europäisches Patentamt**
**European Patent Office**
**Office européen des brevets**

(11) **EP 4 691 636 A1**

(12) **EUROPEAN PATENT APPLICATION**
published in accordance with Art. 153(4) EPC

(43) Date of publication:
**11.02.2026 Bulletin 2026/07**

(21) Application number: 24780081.6

(22) Date of filing: **22.03.2024**

(51) International Patent Classification (IPC):
*B01J 29/48* (2006.01)      *B01J 35/51* (2024.01)
*B01J 37/04* (2006.01)      *B01J 37/08* (2006.01)
*B01J 37/10* (2006.01)      *C01B 39/38* (2006.01)
*C07B 61/00* (2006.01)      *C07C 1/12* (2006.01)
*C07C 15/08* (2006.01)

(52) Cooperative Patent Classification (CPC):
**B01J 29/48; B01J 35/51; B01J 37/04; B01J 37/08;
B01J 37/10; C01B 39/38; C07B 61/00; C07C 1/12;
C07C 15/08;** Y02P 20/52

(86) International application number:
**PCT/JP2024/011505**

(87) International publication number:
**WO 2024/203963 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.03.2023 JP 2023048988**

(71) Applicants:
• **NIPPON STEEL CORPORATION
Chiyoda-ku
Tokyo 100-8071 (JP)**
• **Chiyoda Corporation
Kanagawa 220-8765 (JP)**
• **HighChem Co., Ltd
Tokyo 105-0001 (JP)**

(72) Inventors:
• **Miura, Ayaka
Tokyo 100-8071 (JP)**
• **NAKAO, kenji
Tokyo 100-8071 (JP)**
• **TSUBAKI, Noritatsu
Toyama-shi Toyama 930-8555 (JP)**
• **HIROHATA, Osamu
Yokohama-shi, Kanagawa 220-8765 (JP)**
• **YOSHIMOTO, Ryosuke
Tokyo 105-0001 (JP)**
• **YU, Lin
Tokyo 105-0001 (JP)**

(74) Representative: **Vossius & Partner
Patentanwälte Rechtsanwälte mbB
Siebertstraße 3
81675 München (DE)**

(54) **CATALYST FOR PRODUCING P-XYLENE, METHOD FOR PRODUCING CATALYST FOR PRODUCING P-XYLENE, AND METHOD FOR PRODUCING P-XYLENE**

(57)     Provided are: a catalyst for producing p-xylene, including: a first catalyst containing a ternary composite oxide of chromium, zinc, and zirconium, and a second catalyst containing H-ZSM-5 zeolite coated with an amorphous silicon-containing oxide; a method of producing the same; and a method of producing p-xylene using the same.

EP 4 691 636 A1

## Description

Technical Field

The present disclosure relates to a catalyst for producing p-xylene, a method of producing a catalyst for producing p-xylene, and a method of producing p-xylene.

Background Art

In recent years, interest in global warming has increased. The Conference of the Parties (COP), which discusses an international framework for reducing greenhouse gas emissions and the like, has set a global long-term goal of keeping the increase in average temperature well below 2°C compared to pre-industrial levels, with the aim of limiting emissions peaks as soon as possible and rapidly reducing emissions in accordance with the latest science. The COP21 Paris Agreement states that all countries should strive to formulate and submit long-term low greenhouse gas emissions development strategies. Japan has set a long-term goal of aiming to reduce greenhouse gas emissions by 80% by 2050. Of all the greenhouse gases emitted artificially, carbon dioxide is estimated to have the most significant impact, and efforts to develop technologies to reduce carbon dioxide emissions are being vigorously undertaken in various places.

As one of the countermeasures, several attempts have been proposed to convert emitted carbon dioxide into useful substances, but a large amount of energy is required to convert carbon dioxide into other substances, and there is a need to develop effective catalysts to promote the reaction.

Furthermore, it is necessary to produce useful substances in high demand to attain a technology that contributes to carbon dioxide reduction. p-Xylene is a valuable compound as a raw material for the general-purpose resin polyethylene terephthalate (PET). By making it possible to efficiently produce such compounds from carbon dioxide and hydrogen, a valuable strategy for reducing carbon dioxide emissions would be provided.

Conventionally, p-xylene has been produced by reforming crude oil and naphtha. Furthermore, a technique of producing p-xylene using a mixed gas of carbon monoxide and hydrogen, so-called synthesis gas, as a raw material has been proposed (for example, Non Patent Literature 1, Patent Literature 1, and Patent Literature 3). In the technology disclosed in Non Patent Literature 1, a catalyst having a $ZnCr_2O_4$ spinel structure is used as a catalyst for synthesizing methanol from a synthesis gas, and a catalyst in which zinc-doped H-ZSM-5 zeolite is coated with silicalite-1 is used as a catalyst for synthesizing p-xylene from methanol. Introducing a synthesis gas into a system in the presence of these catalysts makes it possible to synthesize p-xylene in one stage.

In addition to the conventional method of producing p-xylene from carbon monoxide and hydrogen, a method of producing p-xylene from carbon dioxide and hydrogen has also been disclosed.

For example, Patent Literature 2 discloses a "catalyst for producing p-xylene, including a first catalyst containing chromium oxide and a second catalyst containing H-ZSM-5 zeolite coated with silicalite-1," which synthesizes p-xylene in one stage from carbon dioxide and hydrogen. In this technology, a $Cr_2O_3$ catalyst is used as a catalyst for synthesizing methanol from carbon dioxide and hydrogen, and a catalyst in which H-ZSM-5 zeolite is coated with silicalite-1 is used as a catalyst for synthesizing p-xylene from methanol. By mixing these, p-xylene can be synthesized from carbon dioxide and hydrogen in a single stage. Here, the $Cr_2O_3$ catalyst is synthesized by a wet precipitation method, and the H-ZSM-5 is synthesized by hydrothermal synthesis.

Non Patent Literature 1: Peipei Zhang et al., Chemical Science, The Royal Society of Chemistry, October 2017, vol. 8, pp. 7941-7946

Patent Literature 1: Japanese National-Phase Publication (JP-A) No. 2020-535966
Patent Literature 2: Japanese Patent Application Laid-Open (JP-A) No. 2019-205969
Patent Literature 3: JP-A No. 2019-513541

SUMMARY OF INVENTION

Technical Problem

However, to efficiently produce p-xylene using carbon dioxide and hydrogen as raw materials, there is room for improving the catalyst and increasing the productivity and selectivity for p-xylene.

Hence, the present disclosure has been made in consideration of the above problems. An object of the disclosure is to provide a catalyst for producing p-xylene that can efficiently produce p-xylene using carbon dioxide and hydrogen as raw materials, a method of producing a catalyst for producing p-xylene, and a method of producing p-xylene.

Solution to Problem

The gist of the disclosure is as follows.

<1> A catalyst for producing p-xylene, including:

a first catalyst containing a ternary composite oxide of chromium, zinc, and zirconium; and
a second catalyst containing H-ZSM-5 zeolite coated with an amorphous silicon-containing oxide.

<2> The catalyst for producing p-xylene according to <1>, wherein the ternary composite oxide contains chromium at from 20% by mass to 60% by mass, zinc at from 5% by mass to 40% by mass, and zirconium at from 10% by mass to 75% by mass with respect to a total metal amount contained in the first catalyst.
<3> The catalyst for producing p-xylene according to <1> or <2>, wherein the second catalyst is contained at from 10% by mass to 1000% by mass with respect to the first catalyst.
<4> A method of producing p-xylene, including a step of synthesizing p-xylene by bringing carbon dioxide and hydrogen into contact with the catalyst for producing p-xylene according to any one of <1> to <3>.
<5> A method of producing a catalyst for producing p-xylene, which is a method of producing the catalyst for producing p-xylene according to any one of <1> to <3>, the method including:

a step of obtaining a complex containing chromium, zinc, and zirconium by a coprecipitation method or a sol-gel method, and performing one or both of drying and firing the complex, thereby obtaining the first catalyst;
from a silicon compound and an aluminum compound by a hydrothermal synthesis method or by heating TEOS, a step of performing hydrothermal synthesis or heating in the presence of a silicon compound and H-ZSM-5 zeolite, thereby obtaining the second catalyst containing H-ZSM-5 zeolite coated with the amorphous silicon-containing oxide; and
a step of mixing the first catalyst with the second catalyst.

Advantageous Effects of Invention

According to the present disclosure, it is possible to provide a catalyst for producing p-xylene that can efficiently produce p-xylene using carbon dioxide and hydrogen as raw materials, a method of producing a catalyst for producing p-xylene, and a method of producing p-xylene.

BRIEF DESCRIPTION OF DRAWINGS

FIG. 1 is a diagram showing an example of an X-ray diffraction pattern of the first catalyst (ternary composite oxide of Zn, Cr, and Zr).
FIG. 2 is a diagram showing an example of a TEM analysis image of the first catalyst (ternary composite oxide of Zn, Cr, and Zr).
FIG. 3 shows a TEM lattice image of the analyzed spots in (a) in FIG. 2.
FIG. 4 is a diagram showing the results of EDS analysis (energy dispersive X-ray spectroscopy) at the analyzed spots in (a) in FIG. 2.

DESCRIPTION OF EMBODIMENTS

An embodiment that is an example of the disclosure will be described.
In the disclosure, a numerical range expressed using "to" means a range that includes the numerical values before and after "to" as the lower and upper limits.
Concerning the contents of elements contained in the catalyst, "%" means "% by mass" unless otherwise specified.
In the numerical ranges described in stages in the disclosure, the upper limit value of a specific numerical range may be replaced by the upper limit value of another numerical range described in stages or by a value shown in the Examples. In the numerical ranges described in stages in the disclosure, the lower limit value of a specific numerical range may be replaced by the lower limit value of another numerical range described in stages or by a value shown in the Examples.
The term "step" includes not only an independent step but also a step that cannot be clearly distinguished from other steps as long as the intended purpose of the step is achieved.

To efficiently synthesize p-xylene from carbon dioxide and hydrogen, it may be possible to use a Cu/ZnO-based catalyst, which has a high productivity for the intermediate methanol. However, this catalyst has a high hydrogenation ability and hydrogenates the p-xylene produced. Thus, simply using a Cu/ZnO-based methanol synthesis catalyst is impossible.

Moreover, it may also be considered to use a catalyst for synthesizing p-xylene from carbon monoxide and hydrogen, such as that disclosed in Patent Literature 3, which is obtained by supplementing a mixed powder of an active metal oxide such as $ZnCr_2O_4$ and ZSM-5 zeolite with a dispersant such as $ZrO_2$, which serves as a carrier for the active metal oxide. However, in the case of generating p-xylene from carbon dioxide and hydrogen, there are insufficient oxygen defects on the surface of binary active metal oxide particles of $ZnCr_2O_4$ or the like to serve as adsorption sites for carbon dioxide. Therefore, such a catalyst cannot be a highly active catalyst for synthesizing p-xylene from carbon dioxide.

The inventors of the disclosure have found through intensive studies that the productivity and selectivity for p-xylene can be improved by changing the active metal component and structure of a chromium oxide-based catalyst for synthesizing methanol.

As a result of thorough investigation of the constituent elements of the catalyst, it was found that high p-xylene productivity can be obtained by using a ternary composite oxide catalyst in which chromium, zinc, and zirconium are dissolved (hereinafter also referred to as a "ternary composite oxide catalyst containing chromium, zinc, and zirconium," "ternary composite oxide of chromium, zinc, and zirconium," or the like). This has led to the invention of the disclosure.

Preferred embodiments of the disclosure will be described in detail below.

<1. Catalyst for Producing p-Xylene>

First, a preferred embodiment of the catalyst for producing p-xylene of the disclosure will be described. The catalyst for producing p-xylene according to the disclosure catalyzes a synthesis reaction of a hydrocarbon compound, particularly p-xylene, using carbon dioxide and hydrogen as raw materials. The catalyst for producing p-xylene according to the disclosure includes: a first catalyst containing a ternary composite oxide containing chromium, zinc, and zirconium; and a second catalyst containing H-ZSM-5 zeolite coated with an amorphous silicon-containing oxide.

The first catalyst containing a composite oxide containing chromium, zinc, and zirconium catalyzes the conversion of carbon dioxide and hydrogen into methanol. Meanwhile, the second catalyst containing H-ZSM-5 zeolite coated with an amorphous silicon-containing oxide catalyzes the conversion of methanol into p-xylene. In other words, the catalyst for producing p-xylene according to the disclosure is a catalyst composition (composite catalyst) including a first catalyst and a second catalyst with different functions.

(1.1. First Catalyst)

As stated above, the first catalyst is a ternary composite oxide of chromium, zinc, and zirconium. The ternary composite oxide of chromium, zinc, and zirconium catalyzes the conversion of carbon dioxide and hydrogen into methanol. Since the ternary composite oxide containing chromium, zinc, and zirconium has oxygen defects, carbon dioxide is easily adsorbed onto the surface of the composite oxide containing chromium, zinc, and zirconium. The carbon dioxide adsorbed on the composite oxide containing chromium, zinc, and zirconium reacts with hydrogen efficiently, producing methanol efficiently. This catalyst can efficiently produce methanol even when the raw material gas contains carbon monoxide gas, by returning a portion of the gas after the reaction (including unreacted carbon dioxide gas and by-product carbon monoxide gas). In addition, by synthesizing the composite oxide containing chromium, zinc, and zirconium using a sol-gel method, the specific surface area of the catalyst can be increased, and the methanol synthesis reaction from carbon dioxide and hydrogen can be promoted more efficiently, resulting in improved productivity of p-xylene.

The first catalyst may contain other compounds resulting from the production of the first catalyst or compounds derived from the carrier used to support the first catalyst. The mass concentration of chromium with respect to the total metal amount contained in the first catalyst is preferably from 20% by mass to 60% by mass and more preferably from 20% by mass to 55% by mass in a case in which the first catalyst is a ternary composite oxide of chromium, zinc, and zirconium.

The mass concentration of zinc with respect to the total metal amount contained in the first catalyst is preferably from 5% by mass to 40% by mass and more preferably from 20% by mass to 40% by mass.

The mass concentration of zirconium with respect to the total metal amount contained in the first catalyst is preferably from 10% by mass to 75% by mass and more preferably from 30% by mass to 60% by mass.

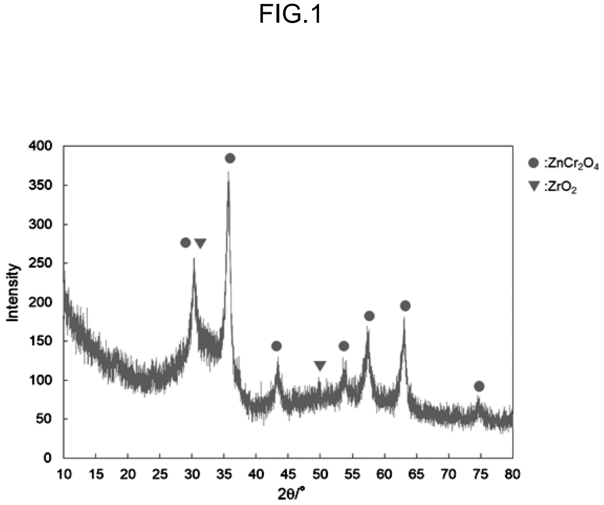

The first catalyst can be identified as a ternary composite oxide from its X-ray diffraction pattern. FIG. 1 shows an example of an X-ray diffraction pattern of the first catalyst (ternary composite oxide of Zn, Cr, and Zr). In the X-ray diffraction pattern shown in FIG. 1, peaks of $ZnCr_2O_4$ and $ZrO_2$ are confirmed, thereby allowing the catalyst to be identified as a ternary composite oxide of Zn, Cr, and Zr.

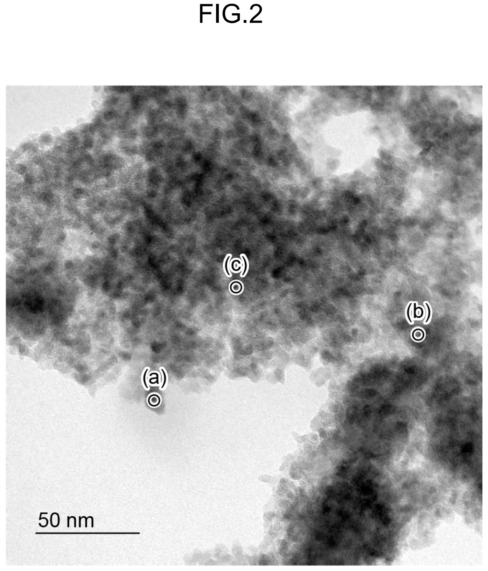

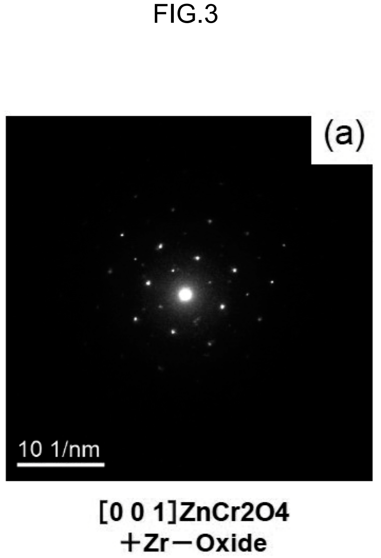

FIG. 2 shows an example of a TEM analysis image of the first catalyst (ternary composite oxide of Zn, Cr, and Zr), and (a) to (c) in the figure show the analyzed spots of the TEM lattice image and EDS. FIG. 3 shows a TEM lattice image of the analyzed spots in (a) in FIG. 2.

From the TEM lattice image shown in FIG. 3, this catalyst is composed of $ZnCr_2O_4$ particles, in which Zr is present in solid solution, with particle sizes on the order of a single nm. For example, this differs from the form shown in Patent Literature 3, in which $ZrO_2$ as a dispersant coexists as separate particles with binary active metal oxide particles of $ZnCr_2O_4$ or the like. The TEM lattice image confirms a clear diffraction lattice image from composite oxide particles of $ZnCr_2O_4$ and a Zr oxide. This shows that the metal oxide consisting of three components of the disclosure has a ternary composite oxide structure (Zr is dissolved in $ZnCr_2O_4$).

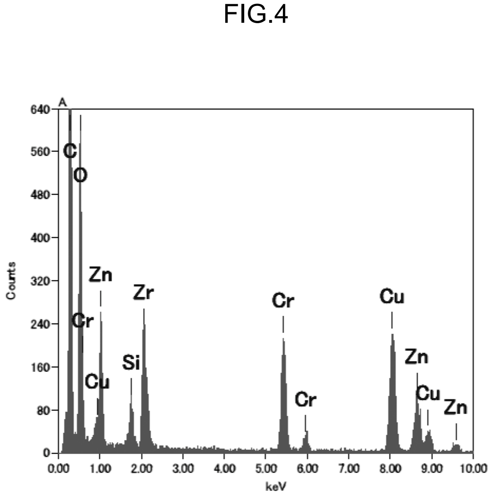

FIG. 4 shows the results of EDS analysis (energy dispersive X-ray spectroscopy) at the analyzed spots in (a) in FIG. 2. From the EDS analysis shown in FIG. 4, the presence of Zn, Cr, and Zr, which are active metal elements constituting the ternary composite oxide according to this catalyst, can be confirmed. It is understood from the analysis results in FIGS. 3 and 4 that the metal oxide consisting of three components according to the disclosure is a ternary composite oxide (solid solution) consisting of Zn, Cr, and Zr.

Furthermore, the metal mass concentration in the first catalyst can be quantified by a scanning inductively coupled plasma atomic emission spectroscopy method (ICP-AES method).

Specifically, a sample is crushed, and an alkali melting agent (such as sodium carbonate or sodium borate) is added thereto. The sample is heated and melted in a platinum crucible. After cooling, the entire amount is dissolved in a hydrochloric acid solution under heating. When the solution is injected into an ICP analyzer, the sample solution is atomized and thermally excited in the high-temperature plasma state inside the equipment, and when it returns to its ground state, an emission spectrum with a wavelength specific to the element is generated. Therefore, the contained elemental species and amounts can be qualitatively and quantitatively determined from the emission wavelength and intensity.

The form of the first catalyst is not particularly limited, and may be, for example, granular. The first catalyst is usually porous, thus providing pores for the reaction to take place, and has a relatively large specific surface area. The specific surface area and the like in a case in which the first catalyst is porous will be described below.

The specific surface area of the first catalyst is not particularly limited, but is, for example, from 3 $m^2$/g to 500 $m^2$/g, and preferably from 10 $m^2$/g to 200 $m^2$/g. This makes it possible to supply a sufficient number of active sites for the reaction of hydrogen with carbon dioxide. Furthermore, by having the specific surface area be equal to or less than the above-described upper limit, it is possible to prevent the pore size from becoming excessively small, resulting in a difference in the gas diffusion rates of carbon dioxide and hydrogen within the pores and a difference in the partial pressure of carbon dioxide and the partial pressure of hydrogen within the first catalyst. As a result, hydrogen and carbon dioxide are efficiently converted into methanol. The specific surface area can be measured by the BET method.

The average pore size of the first catalyst is not particularly limited, but is, for example, from 0.5 nm to 100 nm, and preferably from 2 nm to 30 nm. This prevents the occurrence of a difference in gas diffusion rate between carbon dioxide and hydrogen in the pores. It also makes it possible to increase the specific surface area of the first catalyst and supply a sufficient number of active sites. As a result, hydrogen and carbon dioxide are efficiently converted into methanol.

The pore size can be determined by analyzing data obtained from a gas adsorption method (BET specific surface area measurement) using the Barrett-Joyner-Halenda (BJH) method. The adsorption gas used in the measurement can be, for example, $N_2$ molecules, and the specific surface area can be measured from the amount of adsorption of gas molecules and the weight of the measurement sample. Furthermore, by carrying out this measurement at a temperature at which the adsorbed molecules liquefy, the liquid adsorbed molecules filling the pores can be measured, and the pore volume and the pore size can be determined.

The pore volume of the first catalyst is not particularly limited and is, for example, from 0.1 cc/g to 5 cc/g, and preferably from 0.5 cc/g to 2 cc/g.

The pore volume can be determined by mercury intrusion porosimetry. In a case in which mercury porosimetry is unavailable, it can be measured by a water titration method. The average pore size can be measured by a mercury intrusion method using a mercury porosimeter. In a case in which the mercury intrusion method is unavailable, it can be measured by the gas adsorption method described above.

In a case in which the first catalyst is in a granular form, the average particle size of the first catalyst is, for example, from 1 $\mu$m to 800 $\mu$m, and preferably from 10 $\mu$m to 200 $\mu$m. This accelerates the mass transfer between the first catalyst and the second catalyst, and reduces the flow resistance of the raw material gas (reducing pressure loss). Herein, the "average particle size" refers to the volume-based 50% particle size (D50) measured by a wet laser diffraction/scattering method.

(1.2. Second Catalyst)

As stated above, the second catalyst contains H-ZSM-5 zeolite coated with an amorphous silicon-containing oxide. The second catalyst catalyzes the conversion of methanol produced over the first catalyst into p-xylene. Here, in the second catalyst, the H-ZSM-5 zeolite catalyzes the selective conversion of mainly methanol into p-xylene. Meanwhile, the amorphous silicon-containing oxide coating the H-ZSM-5 zeolite surface prevents the isomerization of p-xylene.

The second catalyst includes, as a main component, H-ZSM-5 zeolite coated with an amorphous silicon-containing oxide. Meanwhile, the amorphous silicon-containing oxide in the second catalyst may contain trace amounts of other elements resulting from the production of the second catalyst or elements derived from the carrier used to support the second catalyst. Other possible elements include aluminum and magnesium.

The amorphous nature of the silicon-containing oxide is determined by observing a broad X-ray diffraction image. In the case of the crystalline silicon-containing oxide, the $SiO_2$ peak intensity appears high, while when it is amorphous, a peak of weak intensity appears, making it possible to distinguish between them. Furthermore, the crystallinity and non-crystallinity can also be determined by an electron diffraction pattern obtained by a transmission electron microscope. In the case of a crystalline material, the structure is periodic, resulting in strong diffraction and a clear electron diffraction pattern. Meanwhile, in the case of an amorphous material, the structure is not periodic, resulting in weak diffraction and a halo-shaped image. Hereinafter, amorphous $SiO_2$ may be simply referred to as "$SiO_2$."

Specifically, many acid sites are present in the 10-membered ring pores of H-ZSM-5 zeolite, and these acid sites act as active sites, thereby forming an aromatic ring (benzene ring) from methanol via dimethyl ether and light olefins. Furthermore, the benzene ring is methylated at its positions 1 and 4 with additional methanol within the pores of the H-ZSM-5 zeolite via the Friedel-Crafts reaction. Here, the 10-membered ring pores of H-ZSM-5 zeolite have a pore size that allows p-xylene to pass through but does not allow o-xylene or m-xylene to pass through. Therefore, p-xylene is preferentially produced due to the so-called spatial confinement effect. The spatial confinement effect refers to a phenomenon or effect in which, when a reaction proceeds within a particular space, the reaction is controlled or confined (restricted) according to the shape and size of the space, and a specific reaction or a reaction at a particular site proceeds preferentially.

Meanwhile, there is no spatial restriction on the outer surface of H-ZSM-5 zeolite, excluding the pores. Thus, the spatial confinement effect does not occur. Therefore, o-xylene, m-xylene, and trimethylbenzene may be produced at the acid points on the outer surface. In contrast, in the disclosure, the surface (outer surface) of H-ZSM-5 zeolite is coated with an amorphous silicon-containing oxide.

The amorphous silicon-containing oxide is an oxide containing silica as a main component, and is more preferably $SiO_2$. $SiO_2$ has low acidity and essentially does not have the function of catalyzing the reaction of methanol or a methanol-derived compound. By coating the outer surface of H-ZSM-5 zeolite with $SiO_2$, which does not contribute to such reactions, the acid sites, which are active sites on the outer surface of H-ZSM-5 zeolite, can be eliminated, and the isomerization of p-xylene to m-xylene and o-xylene and the methylation reaction to trimethylbenzene can be prevented. Meanwhile, p-xylene produced within the pores of H-ZSM-5 zeolite is released to the outside of the second catalyst through the pores of $SiO_2$.

In the second catalyst, the amorphous silicon-containing oxide and H-ZSM-5 zeolite may be in any positional relationship as long as the surface of H-ZSM-5 zeolite is covered with the amorphous silicon-containing oxide. For example, the second catalyst may have a so-called core-shell structure in which particles of H-ZSM-5 zeolite form a core, and the core is covered with an amorphous silicon-containing oxide as a shell.

In the second catalyst, the thickness of the amorphous silicon-containing oxide coating the surface of H-ZSM-5 zeolite is not particularly limited, but is, for example, from 0.01 μm to 200 μm, and preferably from 0.02 μm to 20 μm. This makes it possible to more reliably prevent isomerization of p-xylene to m-xylene and o-xylene, and also to avoid a decrease in reaction efficiency due to an excessively large film thickness of the amorphous silicon-containing oxide, resulting in insufficient supply of methanol to H-ZSM-5 zeolite.

The thickness of the amorphous silicon-containing oxide film formed on the outer surface of H-ZSM-5 zeolite can be measured by observing the cross section of the catalyst using a scanning electron microscope (SEM). One method of preparing a sample for observing the cross-section of a catalyst is to embed catalyst particles in a resin and then polish the resin. Note that the above-described film thickness is an average value. In the case of measuring by observing the catalyst cross-section with an SEM, as long as the film thickness is uniform, several measurement points are sufficient. However, in a case in which the film thickness is not uniform, measurement points are set to a level that allows calculating the average value (e.g., 16 points spaced approximately evenly around the circumference).

Furthermore, in a case in which the film thickness differs for each particle, a plurality of particles (e.g., 10 particles) are observed as representatives and averaged. In selecting a plurality of representative particles, particles having an average film thickness are selected after observing more particles than the representative particles and excluding particles having significantly different film thicknesses. In a case in which catalysts having different particle sizes are mixed, when observing as representative ones as described above, one having a particle size approximately equal to the average particle size is selected. The average particle size is measured using a laser diffraction particle size distribution measuring device, which irradiates dispersed catalyst particles with laser light and determines the particle size distribution by measuring the angle dependency of the scattered light intensity from the particles. There may be some defects where an oxide film containing amorphous silicon is not formed. In such cases, the defects are not included in the measurement points, and the average value of the points where an amorphous silicon-containing oxide is formed is used.

In the second catalyst, the mass ratio of the amorphous silicon-containing oxide is not particularly limited, but is, for example, from 5 % by mass to 100 % by mass, and preferably from 10 % by mass to 40 % by mass with respect to the mass of H-ZSM-5 zeolite. This makes it possible to more reliably prevent isomerization of p-xylene to m-xylene and o-xylene, and also to avoid a decrease in reaction efficiency due to an excessively large film thickness of the amorphous silicon-containing oxide, resulting in insufficient supply of methanol to H-ZSM-5 zeolite.

The mass proportion of the amorphous silicon-containing oxide in the second catalyst is determined by the following method. First, the silica ($SiO_2$ + H-ZSM-5) and alumina in the second catalyst are quantitatively analyzed by a scanning inductively coupled plasma atomic emission spectroscopy method (ICP-AES method). The silica/alumina ratio of H-ZSM-5 is then obtained by cross-sectional analysis using scanning electron microscopeenergy dispersive spectrometry (SEM-EDS). Considering the silica/alumina ratio of H-ZSM-5, the total silica quantified by the ICP-AES method is divided into the amorphous silicon-containing oxide portion and the H-ZSM-5 portion, thereby calculating the mass proportion of the amorphous silicon-containing oxide.

The average pore size of the second catalyst is not particularly limited, but is, for example, from 0.1 nm to 2.5 nm, and preferably from 0.4 nm to 0.6 nm. This makes it possible to obtain the above-mentioned spatial confinement effect within the pores more reliably, and the pore diameter becomes large enough to allow p-xylene synthesized by H-ZSM-5 inside the second catalyst to pass through.

The specific surface area of the second catalyst is not particularly limited, but is, for example, from 20 $m^2$/g to 1000 $m^2$/g, and preferably from 100 $m^2$/g to 400 $m^2$/g. This makes it possible to supply a sufficient number of active sites for the reaction of methanol and the synthesis of p-xylene, and also makes it possible to easily adjust the pore size of the second catalyst to fall within the range described above.

The pore volume of the second catalyst is not particularly limited and is, for example, from 0.1 cc/g to 5 cc/g, and preferably from 0.5 cc/g to 2 cc/g.

In a case in which the second catalyst is in a granular form, the average particle size of the second catalyst can be, for example, from 0.1 $\mu$m to 50 $\mu$m, and preferably from 0.5 $\mu$m to 5 $\mu$m. This accelerates the mass transfer between the 501 catalyst and the second catalyst, and reduces the flow resistance of the raw material gas (reducing pressure loss).

(1.3. Positional Relationship between First Catalyst and Second Catalyst)

As described above, in the disclosure, the catalyst for producing p-xylene includes a first catalyst and a second catalyst. In the disclosure, the expression "the catalyst for producing p-xylene contains a first catalyst and a second catalyst" means that the first catalyst and the second catalyst are present without being physically separated by other elements such as glass wool.

In a case in which the positional relationship between the first catalyst and the second catalyst in the catalyst for producing p-xylene is not particularly limited, for example, the first catalyst and the second catalyst are granular, it can mean that they are physically mixed. Alternatively, in a case in which the first catalyst and the second catalyst each constitute a layer, the layer of the first catalyst and the layer of the second catalyst may be layered on each other. In this case, the amorphous silicon-containing oxide of the second catalyst is disposed on the outer surface side of the catalyst for producing p-xylene. Specifically, the second catalyst may be disposed around the first catalyst.

More preferably, the first catalyst and the second catalyst are brought into proximity. They can be brought into proximity by granulation after physical mixing. The reason is that as long as they are brought into proximity, other side reactions from the intermediate methanol are suppressed, and the p-xylene selectivity is improved.

The second catalyst is contained at, for example, from 10% by mass to 1000% by mass (i.e., the second catalyst accounts for from 10 parts by mass to 1000 parts by mass with respect to 100 parts by mass of the first catalyst), and preferably from 50% by mass to 200% by mass with respect to the first catalyst in the catalyst for producing p-xylene. This allows the methanol synthesis rate in the first catalyst and the methanol consumption rate in the second catalyst to be relatively close, thereby preventing unintended side reactions from occurring due to excess compounds.

By adjusting the masses (compounding amounts) of the first catalyst and the second catalyst during mixing to produce the catalyst for producing p-xylene according to the disclosure, the proportion of the second catalyst to the first catalyst can be optimized. In the catalyst after the first catalyst and the second catalyst are mixed (including a case in which the catalyst is subsequently molded or classified), the ratio can be determined, for example, by using a scanning inductively coupled plasma atomic emission spectroscopy method (ICP-AES method). Specifically, a sample is crushed, and an alkali melting agent (such as sodium carbonate or sodium borate) is added thereto. The sample is heated and melted in a platinum crucible. After cooling, the entire amount is dissolved in a hydrochloric acid solution under heating. When the solution is injected into an ICP analyzer, the sample solution is atomized and thermally excited in the high-temperature plasma state inside the equipment, and when it returns to its ground state, an emission spectrum with a wavelength specific to the element is generated. Therefore, the contained elemental species and amounts can be qualitatively and quantitatively determined from the emission wavelength and intensity. The ratio of the first catalyst to the second catalyst can be calculated considering the ratio of the quantified contained element species.

The catalyst for producing p-xylene according to the disclosure has been described above. According to the disclosure, it is possible to efficiently produce p-xylene using carbon dioxide and hydrogen as raw materials. In other words, as the first catalyst, a ternary composite oxide of chromium, zinc, and zirconium is used, which readily adsorbs carbon dioxide and allows the reaction between carbon dioxide and hydrogen to proceed efficiently, and as the second catalyst, H-ZSM-5 zeolite, which has a spatial confinement effect and can selectively and efficiently synthesize p-xylene, and is coated with an amorphous silicon-containing oxide, is used, thereby allowing p-xylene to be synthesized efficiently and in a high yield. The raw material gas may partially contain carbon monoxide.

<2. Method of Producing Catalyst for Producing p-Xylene>

Next, the method of producing a catalyst for producing p-xylene according to the disclosure will be described.
The method of producing a catalyst for producing p-xylene according to the disclosure is not particularly limited, but, for example, the method includes: a step of performing hydrothermal synthesis or heating in the presence of at least a silicon compound and H-ZSM-5 zeolite, thereby obtaining a second catalyst in which the surface of the H-ZSM-5 zeolite is coated with an amorphous silicon-containing oxide (third step); and a step of mixing the first catalyst containing chromium oxide with the second catalyst (fourth step). The method of producing a catalyst for producing p-xylene according to the disclosure may optionally include, before the third step, a step (second step) of synthesizing H-ZSM-5 zeolite from a silicon compound and an aluminum compound by hydrothermal synthesis. Furthermore, the method of producing a catalyst for producing p-xylene according to the disclosure may optionally include, separately from the second and third steps, a step (first step) of obtaining a complex containing chromium, zinc, and zirconium by a coprecipitation method or a sol-gel method, and drying and/or firing the complex, thereby obtaining a first catalyst containing a ternary composite oxide of chromium, zinc, and zirconium.

(2.1. First Step)

First, in the first step, although not particularly limited, a complex containing a ternary metal of chromium, zinc, and zirconium is obtained by, for example, a coprecipitation method or a sol-gel method, and the complex is dried and/or fired, thereby obtaining a first catalyst.
In the case of the sol-gel method, specifically, first, a chromium compound, a zinc compound, and a zirconium compound are dissolved as metal compounds (metal sources) in a solution of formamide and propylene oxide. The solution is heated at from 50°C to 100°C and allowed to stand for 2 hours or more, thereby obtaining a gel-like colloid.
The metal compounds (metal sources) are not particularly limited, as long as they are soluble in an aqueous solution. For example, metal nitrates, metal acetates, metal chlorides, metal bromides, ammonium salts of heavy metal oxoacids, and the like can be used.
The resulting gel-like colloid is then dried and/or fired, thereby obtaining a first catalyst containing a ternary composite oxide of chromium, zinc, and zirconium.
In the case of the coprecipitation method, specifically, a chromium compound, a zinc compound, and a zirconium compound are first dissolved in an aqueous solution, and a precipitating agent is added, thereby precipitating a composite hydroxide of zinc, chromium, and zirconium.
The chromium compound (chromium source) is not particularly limited, as long as it is soluble in an aqueous solution. For example, chromium nitrate, chromium acetate, chromium chloride, chromium bromide, ammonium dichromate, and the like can be used.
The zinc compound (zinc source) is not particularly limited, as long as it is soluble in an aqueous solution. For example, zinc nitrate, zinc acetate, zinc chloride, zinc bromide, and the like can be used.
The zirconium compound (zirconium source) is not particularly limited, as long as it is soluble in an aqueous solution. For example, zirconium nitrate, zirconium acetate, zirconium chloride, zirconium bromide, and the like can be used.
The precipitating agent is not particularly limited, as long as it can precipitate (deposit) a composite hydroxide of chromium, zinc, and zirconium. For example, ammonium carbonate, sodium carbonate, sodium hydrogen carbonate, urea, potassium carbonate, and the like can be used.
For the purpose of controlling the particle size and shape of the composite hydroxide of the chromium compound, zinc compound, and zirconium compound, an aging treatment may be carried out in a precipitated state of the composite hydroxide of zinc, chromium, and zirconium. The aging treatment can be carried out by, for example, leaving the composite hydroxide to stand for from 30 minutes to 12 hours.
In the above-described treatment, the temperature of the aqueous solution is not particularly limited, but can be, for example, from 30°C to 100°C.
Next, the obtained composite hydroxide of the chromium compound, zinc compound, and zirconium compound is dried and/or fired, thereby obtaining a first catalyst containing a composite hydroxide of zinc, chromium, and zirconium. Before drying and firing, the composite hydroxide of zinc, chromium, and zirconium may be washed, as appropriate.

Drying can be carried out, for example, in an air atmosphere at from 100°C to 200°C for from 30 minutes to 12 hours. Alternatively, drying can be carried out using supercritical carbon dioxide at from 50°C to 200°C for from 20 minutes to 24 hours. Firing can be carried out, for example, in an air atmosphere at from 400°C to 600°C for from 30 minutes to 10 hours.

As stated above, the first catalyst containing a composite oxide of chromium, zinc, and zirconium is obtained.

(2.2. Second Step)

In this step, H-ZSM-5 zeolite is synthesized from a silicon compound and an aluminum compound by a hydrothermal synthesis method. The formation of H-ZSM-5 zeolite is carried out, for example, by heating an aqueous solution (precursor solution) containing a silicon compound (silica source) and an aluminum compound (alumina source).

Examples of the silicon compound that can be used include tetramethyl orthosilicate and tetraethyl orthosilicate, and examples of the aluminum compound that can be used include aluminum nitrate and aluminum acetate, but are not limited to these.

The precursor solution may also contain an amine compound, such as tetrapropylammonium hydroxide or tetraethylammonium hydroxide, as a template agent (organic structure-directing agent). Furthermore, the solvent of the precursor solution contains water as a main component. Still, it may contain an alcohol solvent such as ethanol or methanol for the purpose of controlling the hydrolysis rate of the silicon compound.

The temperature in hydrothermal synthesis is not particularly limited, but may be, for example, from 150°C to 200°C, and preferably from 170°C to 190°C.

With the increase in hydrothermal synthesis time, the particle size of H-ZSM-5 zeolite increases. The hydrothermal synthesis time is, for example, from 1 to 168 hours, and preferably from 24 to 72 hours.

In hydrothermal synthesis, the reaction liquid may be stirred, as necessary. The stirring conditions can be set, as appropriate. Moreover, the particle size, pore size, specific surface area, and other physical properties of H-ZSM-5 zeolite can be controlled by the hydrothermal synthesis conditions, such as the time.

The H-ZSM-5 zeolite produced by hydrothermal synthesis is washed, if appropriate, and dried after completion of hydrothermal synthesis. The drying time is not particularly limited, but may be, for example, from 0.5 to 20 hours. The drying temperature is not particularly limited, but may be, for example, from 50°C to 150°C.

The dried catalyst is then subjected to a firing treatment, thereby removing any organic matter present within the pores of zeolite. Accordingly, H-ZSM-5 zeolite is obtained. The firing time is not particularly limited, but may be, for example, from 0.5 to 15 hours. The firing temperature is not particularly limited, but may be, for example, from 400°C to 600°C.

This step may be omitted as long as H-ZSM-5 zeolite is available separately. Furthermore, regardless of the above explanation, the H-ZSM-5 zeolite does not have to be produced by a hydrothermal synthesis method.

(2.3. Third Step)

In this step, hydrothermal synthesis is carried out in the presence of at least a silicon-containing compound and H-ZSM-5 zeolite, or heating is carried out in the presence of a silicon-containing compound and H-ZSM-5 zeolite, thereby obtaining a second catalyst in which the surface of the H-ZSM-5 zeolite is coated with an amorphous silicon-containing oxide (preferably $SiO_2$).

The formation of $SiO_2$ is carried out, for example, by dropping tetraethoxysilane (TEOS) into an organic solvent in which H-ZSM-5 zeolite is dispersed, followed by heating.

In addition, hexane or the like is used as the organic solvent in which H-ZSM-5 is dispersed during the $SiO_2$ coating. In addition, other organic solvents, alcohols, and the like may be contained.

Stirring is performed so as to disperse the H-ZSM-5 in the organic solvent, but the stirring speed is not particularly limited. For example, the stirring speed may be set to from 200 to 1000 rpm/min, and preferably from 300 to 800 rpm/min.

The heating temperature is not limited after dropping TEOS into the H-ZSM-5-dispersed organic solvent. For example, the temperature may be from 50°C to 150°C, and preferably from 50°C to 80°C.

TEOS is dropped into the H-ZSM-5-dispersed organic solvent, then the organic solvent is removed by heating. During that, the heating time is not particularly limited, but may be, for example, from 50°C to 150°C, and preferably from 50°C to 80°C.

If necessary, the H-ZSM-5 zeolite coated with $SiO_2$ is subjected to drying and firing after the heating is completed. The drying time is not particularly limited, but may be, for example, from 0.5 to 20 hours. The drying temperature is not particularly limited, but may be, for example, from 50°C to 150°C.

The dried catalyst is then subjected to a firing treatment, thereby removing any organic matter present within the pores of zeolite. Accordingly, $SiO_2$-coated H-ZSM-5 zeolite is obtained. The firing time is not particularly limited, but may be, for example, from 0.5 to 15 hours. The firing temperature is not particularly limited, but may be, for example, from 400°C to 600°C.

(2.4. Fourth Step)

A first catalyst containing a ternary composite oxide of chromium, zinc, and zirconium is physically mixed with a second catalyst containing H-ZSM-5 zeolite coated with an amorphous silicon-containing oxide. Accordingly, a catalyst for producing p-xylene is obtained. Mixing can be carried out, for example, using a mortar, a ball mill, an automatic kneader, or the like.

After mixing, to improve the adhesion between the first catalyst and the second catalyst, the catalyst for producing p-xylene may be molded into pellets, and the pellets may be crushed.

<3. Method of Producing p-Xylene>

Next, the method of producing p-xylene using the above-described catalyst for producing p-xylene will be described based on the preferred embodiment.

Producing p-xylene can be carried out by bringing carbon dioxide and hydrogen into contact with the catalyst for producing p-xylene according to the disclosure. Carbon dioxide and hydrogen may be supplied separately, but they are usually supplied as a mixed gas.

Regarding productivity, a mixed gas in which the total of carbon dioxide and hydrogen is 50% by volume or more of the total of the mixed gas is preferably used as the mixed gas of carbon dioxide and hydrogen used in the method according to the disclosure. The molar ratio of hydrogen to carbon dioxide (hydrogen/carbon dioxide) is particularly preferable in a range of from 0.5 to 4.0. This is because in a case in which the molar ratio of hydrogen to carbon dioxide is less than 0.5, the amount of hydrogen present in the raw material gas is excessively small such that the hydrogenation reaction of carbon dioxide does not proceed easily, and productivity does not increase, while on the other hand, in a case in which the molar ratio of hydrogen to carbon dioxide exceeds 4.0, the amount of carbon dioxide present in the raw material gas is excessively small such that the productivity of liquid hydrocarbons does not increase regardless of the catalytic activity.

The reactor used for bringing the mixed gas into contact with the catalyst according to the disclosure is not particularly limited, and examples thereof include general reactors for gas phase synthesis processes, such as fixed beds, entrained beds, and fluidized beds, reactors for liquid phase synthesis processes, such as slurry beds, and microchannel reactors.

When the reaction for producing p-xylene is carried out, the ternary composite oxide of chromium, zinc, and zirconium (first catalyst) needs to be in a reduced state. Therefore, before the mixed gas is supplied for producing p-xylene, a reducing gas such as hydrogen gas is passed through so as to reduce the catalyst for producing p-xylene. Such reduction treatment is not particularly limited, but can be carried out, for example, at a temperature of from 300°C to 500°C for from 1 to 40 hours.

The catalyst for producing p-xylene may be reduced after or before being loaded into the reactor. For example, it is also possible to carry out a reduction treatment before charging the catalyst for producing p-xylene into the reactor, and then to load the catalyst into the reactor.

The conditions for producing p-xylene are not particularly limited, and the conditions can be set depending on the type of reactor.

For example, the reaction temperature during the reaction for producing p-xylene is not particularly limited, but is from 200°C to 500°C, preferably from 300°C to 400°C. The pressure in the system during the reaction is not particularly limited, but is, for example, from 0.8 to 6.0 MPa, and preferably from 2.5 to 5.0 MPa.

In a case in which activity is reduced due to factors such as an extremely high conversion rate or a long reaction time, the catalyst for producing p-xylene can be regenerated by supplying a gas containing hydrogen (regeneration gas) instead of the mixed gas. The hydrogen content in the regeneration gas is preferably 5% or more. The hydrogen content in the regeneration gas may be 100%. The regeneration gas may contain, in addition to hydrogen, an inert gas such as nitrogen or argon.

The conditions for regenerating the catalyst for producing p-xylene are not particularly limited as long as the catalyst regeneration proceeds. The mechanism of catalyst regeneration by bringing the hydrogen-containing regeneration gas into contact with the catalyst for producing p-xylene is presumably due to the re-reduction of a chromium oxide catalyst or the like that has been oxidized by the by-product water, and the removal of deposited carbon by hydrogen.

Specifically, the temperature during regeneration is, for example, from 300°C to 500°C. The pressure during regeneration is, for example, from ordinary pressure to reaction pressure. In particular, when the regeneration pressure is set to the reaction pressure or lower, it becomes possible to use a compressor to increase the pressure to the reaction pressure in the reaction, and there is no need to install a new compressor for regeneration, which is advantageous in terms of equipment costs. Moreover, the regeneration time can be, for example, 1 hour or more.

EXAMPLES

The catalyst for producing p-xylene and the like according to the disclosure will be described in further detail below with reference to Examples and Comparative Examples; however, the disclosure is not limited to these Examples and Comparative Examples.

[Example 1]

(1. Production of Catalyst for Producing p-Xylene)

Zinc nitrate, chromium nitrate, and zirconium nitrate were precisely weighed at 6.4 g, 8.6 g, and 5.7 g, respectively, and dissolved in 32 g of a solution of formamide and propylene oxide. This solution was further dissolved in 75 mL of water and 225 mL of ethanol. The obtained aqueous solution was allowed to stand at 70°C for 2 hours. Drying was carried out thereafter using supercritical carbon dioxide at 160°C for 6 hours. The obtained solid was coarsely crushed and then fired (calcined) in air at 500°C for 3 hours. As a result, a composite oxide powder of chromium, zinc, and zirconium was obtained as a first catalyst. The obtained composite oxide powder of chromium, zinc, and zirconium had a BET specific surface area of 186 $m^2$/g measured by $N_2$ adsorption/desorption and an average pore size of 3.0 nm.
To prepare H-ZSM-5 zeolite, tetramethyl orthosilicate, aluminum nitrate, water, tetrapropylammonium hydroxide, and ethanol were precisely weighed and dissolved in a molar ratio of 1:1/40:50:0.24:4. The solution was introduced into a Teflon (registered trademark) sealed autoclave, stirred with a magnetic stirrer for 4 hours, and then reacted at 180°C for 24 hours. The product thus obtained was washed with distilled water, dried overnight at 120°C, and then fired (calcined) in air at 550°C for 5 hours so as to decompose and remove the organic matter present in the zeolite pores, thereby obtaining an H-ZSM-5 zeolite powder. The obtained H-ZSM-5 zeolite powder had a BET specific surface area of 367.6 $m^2$/g measured by $N_2$ adsorption/desorption and an average pore size of 2.0 nm.
The thus obtained zeolite (H-ZSM-5) was added at 1 g to 7 g of n-hexane (100 mL) and stirred at 500 rpm/min using a stirrer. Then, 3.4 g of TEOS was added dropwise to the solvent. After the TEOS was added dropwise, stirring was continued for 4 hours. The solution was then heated to 80°C with stirring, and n-hexane was removed by suction. The resulting sample was dried at 100°C for 12 hours and then fired at 550°C for 5 hours, thereby obtaining H-ZSM-5 zeolite coated with amorphous $SiO_2$ (hereinafter also referred to as "$SiO_2$-coated zeolite") as a second catalyst. The obtained $SiO_2$-coated zeolite powder had a BET specific surface area of 308.0 $m^2$/g measured by $N_2$ adsorption/desorption and an average pore size of 2.1 nm. Hereinafter, the second catalyst may be abbreviated as "H-ZSM-5+$SiO_2$."
The thus obtained ternary composite oxide of zinc, chromium, and zirconium (first catalyst) and the $SiO_2$-coated zeolite (second catalyst) were precisely weighed in a mass ratio of 1:2, and physically mixed in a mortar for 10 minutes, thereby obtaining a mixed powder. Thereafter, the mixed powder was press-molded into 3-mmφ tablets using a compression molding machine, thereby obtaining tablet-shaped bodies, which were then crushed and classified to have a size of 20 to 40 mesh. Thus, a catalyst for producing p-xylene was obtained.

(Production of p-Xylene)

The obtained catalyst for producing p-xylene was used in an amount of 0.1 g and fixed with quartz wool, whereby the catalyst was located at the center of a SUS reaction tube having an inner diameter of 6 mm. A thermocouple was inserted at the center of the catalyst layer, and the fixed bed reaction tube was set at a predetermined position.
Before starting the synthesis reaction, the reactor was first heated to 400°C in a nitrogen atmosphere, and then reduction treatment was carried out for 2 hours while flowing hydrogen gas at 60 mL/min.
The temperature was then lowered to room temperature, and a gas containing hydrogen: $CO_2$ = 3:1 and 3% argon as an internal standard was introduced at 40 mL/min, and synthesis was carried out at 5 MPa and 360°C for 4 hours. After removing moisture, the reaction product was injected into two gas chromatographs (GC-8A manufactured by SHIMADZU CORPORATION) and analyzed using a thermal conductivity detector (TCD) and a flame ionization detector (FID).
For the degree of reaction in synthesis reaction (production rate of p-xylene), the $CO_2$ conversion rate, CO selectivity, methane selectivity, C2-C4 (O) selectivity from ethane, propane, and butane, C2-C4 (=) selectivity from ethylene, propylene, and butylene, $CH_3OH$+DME selectivity from methanol and dimethyl ether, selectivity for long-chain aliphatic hydrocarbons from pentane onward (C5+), and aromatics selectivity as well as benzene (B) selectivity, toluene (T) selectivity, o-xylene (o-X) selectivity, m-xylene (m-X) selectivity, p-xylene (p-X) selectivity, selectivity for aromatic hydrocarbons having 9 carbon atoms (A (C9)), and selectivity for aromatic hydrocarbons having 10 or more carbon atoms (A (C10+)) in the aromatic compound, and p-X/X as the proportion of p-xylene in total xylene (o-xylene, m-xylene, and p-xylene), were calculated from the concentration of each component using the following Formulas. The results are shown in Example 1 in Table 1.

$CO_2$ conversion rate (%) = (1- (molar quantity of carbon derived from $CO_2$ after reaction)/(molar quantity of carbon derived from supplied $CO_2$)) $\times$ 100

CO selectivity (%) = (molar quantity of carbon derived from CO)/((molar quantity of carbon derived from supplied $CO_2$) - (molar quantity of carbon derived from $CO_2$ after reaction))) $\times$ 100

Hydrocarbon selectivity (%) = (molar quantity of carbon derived from hydrocarbons)/((molar quantity of carbon derived from supplied $CO_2$) - (molar quantity of carbon derived from $CO_2$ after reaction))) $\times$ 100

$CH_4$ selectivity (%) = (molar quantity of carbon derived from $CH_4$)/(molar quantity of carbon derived from hydrocarbons) $\times$ 100

Ethane selectivity (%) = (molar quantity of carbon derived from ethane)/(molar quantity of carbon derived from hydrocarbons) $\times$ 100

Propane selectivity (%) = (molar quantity of carbon derived from propane)/(molar quantity of carbon derived from hydrocarbons) $\times$ 100

Butane selectivity (%) = (molar quantity of carbon derived from butane)/(molar quantity of carbon derived from hydrocarbons) $\times$ 100

C2-C4 (O) selectivity (%) = (ethane selectivity) + (propane selectivity) + (butane selectivity) Ethylene selectivity (%) = (molar quantity of carbon derived from ethylene)/(molar quantity of carbon derived from hydrocarbons) $\times$ 100

Propylene selectivity (%) = (molar quantity of carbon derived from propylene)/(molar quantity of carbon derived from hydrocarbons) $\times$ 100

Butylene selectivity (%) = (molar quantity of carbon derived from butylene)/(molar quantity of carbon derived from hydrocarbons) $\times$ 100

C2-C4 (=) selectivity (%) = (ethylene selectivity) + (propylene selectivity) + (butylene selectivity)

$CH_3OH$ selectivity (%) = (molar quantity of carbon derived from $CH_3OH$)/(molar quantity of carbon derived from hydrocarbons) $\times$ 100

DME selectivity (%) = (molar quantity of carbon derived from DME)/(molar quantity of carbon derived from hydrocarbons) $\times$ 100

$$CH_3OH+DME \text{ selectivity (\%)} = (CH_3OH \text{ selectivity}) + (DME \text{ selectivity})$$

Pentane selectivity (%) = (molar quantity of carbon derived from pentane)/(molar quantity of carbon derived from hydrocarbons) $\times$ 100

Long-chain aliphatic hydrocarbons from pentane onward (C5+) (%) = (pentane selectivity) + (selectivity for long-chain aliphatic hydrocarbon (C6+)) B selectivity (%) = (molar quantity of carbon derived from benzene)/(molar quantity of carbon derived from hydrocarbons) $\times$ 100

T selectivity (%) = (molar quantity of carbon derived from toluene)/(molar quantity of carbon derived from hydrocarbons) $\times$ 100

E selectivity (%) = (molar quantity of carbon derived from ethylbenzene)/(molar quantity of carbon derived from hydrocarbons) $\times$ 100

o-X selectivity (%) = (molar quantity of carbon derived from o-xylene)/(molar quantity of carbon derived from hydrocarbons) $\times$ 100

m-X selectivity (%) = (molar quantity of carbon derived from m-xylene)/(molar quantity of carbon derived from hydrocarbons) $\times$ 100

p-X selectivity (%) = (molar quantity of carbon derived from p-xylene)/(molar quantity of carbon derived from hydrocarbons) $\times$ 100

A (C9) selectivity (%) = (molar quantity of carbon derived from cumene)/(molar quantity of carbon derived from hydrocarbons) $\times$ 100

Tetralin selectivity (%) = (molar quantity of carbon derived from tetralin)/(molar quantity of carbon derived from hydrocarbons) $\times$ 100

A (C10+) selectivity (%) = (tetralin selectivity) + (selectivity for aromatic hydrocarbon of C11 or higher) Aromatics selectivity (%) = (B selectivity) + (T selectivity) + (o-X selectivity) + (m-X selectivity) + (p-X selectivity) + (A (C9) selectivity) + (A (C10+) selectivity) Proportion of p-xylene in total xylene (%) = (p-X selectivity)/((o-X selectivity) + (m-X selectivity) + (p-X selectivity) $\times$ 100

[Example 2]

Zinc nitrate, chromium nitrate, and zirconium nitrate were precisely weighed at 6.4 g, 8.6 g, and 5.7 g, respectively, and dissolved in 100 mL of pure water. To this aqueous solution, an aqueous ammonium carbonate solution was added while heating at 70°C so as to precipitate a ternary composite hydroxide of zinc, chromium, and zirconium, and the mixture was thoroughly stirred with a stirrer. Thereafter, the mixture was aged for 3 hours with stirring while being maintained at 70°C, and then filtered and thoroughly washed with pure water at 80°C. The obtained solid was coarsely crushed and then fired (calcined) in air at 500°C for 3 hours. As a result, a composite oxide powder of chromium, zinc, and zirconium was obtained as a first catalyst, thereby obtaining a catalyst for producing p-xylene. p-Xylene was synthesized in the same manner as in Example 1 using the obtained catalyst for producing p-xylene. The results are shown in Example 2 in Table 1.

[Examples 3 to 5]

A catalyst for producing p-xylene was obtained in the same manner as in Example 1, except that the amounts of zinc nitrate, chromium nitrate, and zirconium nitrate were changed such that the mass ratios of metals contained in the resulting composite oxide were Zn/Cr/Zr = 19/30/52, 25/40/35, and 21/50/29. p-Xylene was synthesized in the same manner as in Example 1 using each obtained catalyst for producing p-xylene. The results are shown in Examples 3 to 5 in Table 1.

[Comparative Example 1]

A catalyst for producing p-xylene was obtained in the same manner as in Example 1, except that chromium oxide ($Cr_2O_3$) was synthesized by wet precipitation as in Patent Literature 2. p-Xylene was synthesized in the same manner as in Example 1 using the obtained catalyst for producing p-xylene. The results are shown in Comparative Example 1 in Table 1.

[Comparative Example 2]

The obtained zeolite (H-ZSM-5) was used in an amount of 1 g and immersed in a solution of silica, tetrapropylammonium hydroxide, ethanol, and water in a molar ratio of 1:0.06:16:240, and then placed in a Teflon-sealed autoclave and reacted at 180°C for 24 hours while rotating at a speed of 3 revolutions per minute. This procedure was repeated twice, thereby obtaining zeolite coated with silicalite-1 as the second catalyst. A catalyst for producing p-xylene was obtained in the same manner as in Example 1, except that this silicalite-1-coated zeolite (H-ZSM-5 + silicalite-1) was used. p-Xylene was synthesized in the same manner as in Example 1 using the obtained catalyst for producing p-xylene. The results are shown in Comparative Example 2 in Table 1.

[Comparative Example 3]

A catalyst for producing p-xylene was obtained in the same manner as in Example 1, except that a zinc-zirconium binary composite oxide was prepared using 0.8 g of zinc nitrate and 5.7 g of zirconium nitrate. p-Xylene was synthesized in the same manner as in Example 1 using the obtained catalyst for producing p-xylene. The results are shown in Comparative Example 3 in Table 1.

[Reference Example 1]

A catalyst for producing p-xylene was obtained in the same manner as in Example 1, except that the upper stage of the reactor was filled with the ternary composite oxide of zinc, chromium, and zirconium prepared in Example 1, and the lower stage was filled with silicalite-coated zeolite (second catalyst) via glass wool. p-Xylene was synthesized in the same manner as in Example 1 using the obtained catalyst for producing p-xylene. The results are shown in Reference Example 1 in Table 1.

[Table 1]

| | Catalyst composition First catalyst/Second catalyst (Metal mass ratio of first catalyst) | $CO_2$ conversion rate [%] | CO Selectivity [%] | p-Xylene selectivity [%] | Space time yield [$g_{PX}$/h·$kg_{cat}$] |
|---|---|---|---|---|---|
| Example 1 | ZnCrZr/H-ZSM-5+$SiO_2$ (Zn/Cr/Zr=31/25/44) | 6.6 | 23.5 | 16.3 | 25.4 |
| Example 2 | ZnCrZr/H-ZSM-5+$SiO_2$ (Zn/Cr/Zr=31/25/44) | 5.3 | 41.4 | 12.9 | 12.6 |
| Example 3 | ZnCrZr/H-ZSM-5+$SiO_2$ (Zn/Cr/Zr=19/30/52) | 6.1 | 61.7 | 19.2 | 14.4 |
| Example 4 | ZnCrZr/H-ZSM-5+$SiO_2$ (Zn/Cr/Zr=25/40/35) | 5.8 | 73.4 | 22.1 | 11.0 |
| Example 5 | ZnCrZr/H-ZSM-5+$SiO_2$ (Zn/Cr/Zr=21/50/29) | 6.0 | 76.4 | 24.1 | 13.5 |
| Comparative Example 1 | $Cr_2O_3$/H-ZSM-5+$SiO_2$ | 2.8 | 39.1 | 14.4 | 7.9 |
| Comparative Example 2 | ZnCrZr/H-ZSM-5+Silicalite-1 (Zn/Cr/Zr=31/25/44) | 5.0 | 70.6 | 9.5 | 4.2 |
| Comparative Example 3 | ZnZr/H-ZSM-5+$SiO_2$ (Zn/Zr=8/92) | 7.8 | 31.2 | 12.3 | 20.2 |

(continued)

| | Catalyst composition First catalyst/Second catalyst (Metal mass ratio of first catalyst) | $CO_2$ conversion rate [%] | CO Selectivity [%] | p-Xylene selectivity [%] | Space time yield [$g_{PX}$/h·kg$_{cat}$] |
|---|---|---|---|---|---|
| Reference Example 1 | ZnCrZr‖H-ZSM-5+$SiO_2$ (Zn/Cr/Zr=31/25/44) | 6.1 | 56.2 | 0.6 | 0.6 |

From the Examples, Comparative Example 1, and Reference Example 1 in Table 1, the Examples in which the first catalyst was a ternary composite oxide of chromium, zinc, and zirconium have higher space time yields than Comparative Example 1 in which chromium oxide was used as the first catalyst and Reference Example 1 in which the first catalyst and the second catalyst were separated by glass wool, and thus show higher p-xylene productivity. Moreover, from the Examples and Reference Example 1 in Table 1, it is understood that the first catalyst and the second catalyst are preferably near each other by physical mixing.

From the Examples and Comparative Example 2 in Table 1, it is also understood that the catalyst for producing p-xylene of the disclosure has higher p-xylene productivity when $SiO_2$-coated H-ZSM-5 is used than when silicalite-1-coated H-ZSM-5 is used, which is preferable.

From the Examples and Comparative Example 3 in Table 1, it is also understood that the catalyst for producing p-xylene of the disclosure exhibits higher p-xylene selectivity than the case in which a binary catalyst of Zn and Zr is used as the first catalyst, and is effective for synthesizing p-xylene with high selectivity.

Although the preferred embodiment of the disclosure has been described in detail above, the disclosure is not limited to such examples. It is clear that a person with ordinary knowledge in the art to which the disclosure pertains can conceive of various modified or altered examples within the scope of the technical ideas described in the claims, and these are naturally understood to fall within the technical scope of the disclosure.

The disclosure of Japanese Patent Application No. 2023-048988, filed on March 24, 2023, is incorporated herein by reference in its entirety. All publications, patent applications, and standards mentioned herein are incorporated herein by reference to the same extent as if each individual publication, patent application, or standard were specifically and individually indicated to be incorporated by reference.

**Claims**

1.    A catalyst for producing p-xylene, comprising:

a first catalyst containing a ternary composite oxide of chromium, zinc, and zirconium; and
a second catalyst containing H-ZSM-5 zeolite coated with an amorphous silicon-containing oxide.

2.    The catalyst for producing p-xylene according to claim 1, wherein the ternary composite oxide contains chromium at from 20% by mass to 60% by mass, zinc at from 5% by mass to 40% by mass, and zirconium at from 10% by mass to 75% by mass, with respect to a total metal amount contained in the first catalyst.

3.    The catalyst for producing p-xylene according to claim 1, wherein the second catalyst is contained at from 10% by mass to 1000% by mass, with respect to the first catalyst.

4.    A method of producing p-xylene, comprising a step of synthesizing p-xylene by bringing carbon dioxide and hydrogen into contact with the catalyst for producing p-xylene according to any one of claims 1 to 3.

5.    A method of producing a catalyst for producing p-xylene, which is a method of producing the catalyst for producing p-xylene according to any one of claims 1 to 3, the method comprising:

a step of obtaining a complex containing chromium, zinc, and zirconium by a precipitation method or a sol-gel method, and performing one or both of drying and firing the complex, thereby obtaining the first catalyst;
a step of performing hydrothermal synthesis or heating in the presence of a silicon compound and H-ZSM-5 zeolite, thereby obtaining the second catalyst containing H-ZSM-5 zeolite coated with the amorphous silicon-containing oxide; and
a step of mixing the first catalyst with the second catalyst.

FIG.1

FIG.2

FIG.3

[0 0 1]ZnCr2O4
+Zr－Oxide

# FIG.4

## INTERNATIONAL SEARCH REPORT

| International application No. |
|---|
| **PCT/JP2024/011505** |

### A. CLASSIFICATION OF SUBJECT MATTER

*B01J 29/48*(2006.01)i; *B01J 35/51*(2024.01)i; *B01J 37/04*(2006.01)i; *B01J 37/08*(2006.01)i; *B01J 37/10*(2006.01)i; *C01B 39/38*(2006.01)i; *C07B 61/00*(2006.01)i; *C07C 1/12*(2006.01)i; *C07C 15/08*(2006.01)i
FI: B01J29/48 M; B01J37/08; B01J37/10; B01J35/51; B01J37/04 101; C01B39/38; C07C15/08; C07C1/12; C07B61/00 300

According to International Patent Classification (IPC) or to both national classification and IPC

### B. FIELDS SEARCHED

Minimum documentation searched (classification system followed by classification symbols)

B01J29/48; B01J35/51; B01J37/04; B01J37/08; B01J37/10; C01B39/38; C07B61/00; C07C1/12; C07C15/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JSTChina/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | CN 110743606 A (DALIAN INSTITUTE OF CHEMICAL PHYSICS, CHINESE ACADEMY OF SCIENCES) 04 February 2020 (2020-02-04) claims, paragraphs [0187], [0367] | 1-5 |
| A | CN 110496640 A (DALIAN INSTITUTE OF CHEMICAL PHYSICS, CHINESE ACADEMY OF SCIENCES) 26 November 2019 (2019-11-26) claims, paragraphs [0081], [0094], [0109] | 1-5 |
| A | CN 107971026 A (CHINA PETROLEUM AND CHEMICAL CORP.) 01 May 2018 (2018-05-01) | 1-5 |
| A | WO 2022/230467 A1 (CHIYODA CORPORATION) 03 November 2022 (2022-11-03) | 1-5 |
| A | CN 113277923 A (DALIAN INSTITUTE OF CHEMICAL PHYSICS, CHINESE ACADEMY OF SCIENCES) 20 August 2021 (2021-08-20) | 1-5 |
| A | JP 2019-205969 A (NIPPON STEEL CORPORATION) 05 December 2019 (2019-12-05) | 1-5 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **14 May 2024** | **28 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

International application No.

**PCT/JP2024/011505**

| Patent document cited in search report | | | Publication date (day/month/year) | Patent family member(s) | | | Publication date (day/month/year) |
|---|---|---|---|---|---|---|---|
| CN | 110743606 | A | 04 February 2020 | (Family: none) | | | |
| CN | 110496640 | A | 26 November 2019 | WO | 2019/218489 | A1 | |
| CN | 107971026 | A | 01 May 2018 | (Family: none) | | | |
| WO | 2022/230467 | A1 | 03 November 2022 | EP | 4119530 | A1 | |
| | | | | CN | 117222610 | A | |
| | | | | CA | 3217855 | A1 | |
| CN | 113277923 | A | 20 August 2021 | (Family: none) | | | |
| JP | 2019-205969 | A | 05 December 2019 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020535966 A
- JP 2019205969 A
- JP 2019513541 A
- JP 2023048988 A

**Non-patent literature cited in the description**

- **PEIPEI ZHANG et al.** Chemical Science. The Royal Society of Chemistry, October 2017, vol. 8, 7941-7946